# EUROPEAN PATENT APPLICATION

(11) **EP 4 000 605 A2**
(43) Date of publication of application: **25.05.2022**
(21) Application number: 20839714.1
(22) Date of filing: 13.07.2020
(51) Int. Cl.: A61K 9/28, A61K 9/30, A61K 9/36, A61K 9/50

(54) **COMPOSITIONS OF FILM COATINGS FOR TABLETS WITH INCREASED GLOSS, METHOD FOR PRODUCTION THEREOF AND APPLICATION OF SAME**

(30) Priority: 15.07.2019 MX 2019008440
(71) Applicant: DVA Farma Mexicana S.A. de C.V., Estado de Hidalgo, 42970 (MX)
(72) Inventor: ESCORCIA RODRÍGUEZ, Francisco, Ciudad de México, 06720 (MX); GARCIA MONDRAGON, Maria de Lourdes, México, 50900 (MX); FLORES NAVARRETE, Ana Karen, Estado de México, 55628 (MX); ESPITIA RODRIGUEZ, José Luis, Ciudad de México, 15210 (MX)
(74) Representative: Clarke Modet & Co.
(86) International application number: PCT/MX2020/050017
(87) International publication number: WO 2021/010814

(57) **Abstract**

The present document discloses compositions and procedures for applying pharmaceutical tablet coatings, recognized as film coatings wherein it is possible to obtain a final product coated with brightness superior to that achievable with typical compositions. The main difference in the composition is in the inclusion of the product derived from isomaltulose, which is a mixture of 6-O-α-D-glucopyranosyl-D-mannitol dihydrate with 6-O-α-D-glucopyranosyl-D- sorbitol, known as Isomalt, combined with a variety of film-forming polymers such as ethylene glycol and polyvinyl alcohol copolymer, copovidone, polyvinyl alcohol, and cellulosic derivatives, wherein the process conditions are advantageous as it is possible to handle high concentrations of solids in their preparation and wider applying temperature ranges than normally recommended for film coatings.

## Description

### FIELD OF THE INVENTION

The present invention relates to compositions based on isomaltulose derivatives, to obtain film-shaped coatings for tablets, supplements, pills, lozenges, dragees, caplets or microspheres, used in the food or pharmaceutical industry which have the feature of increased brightness.

### BACKGROUND OF THE INVENTION

Coatings for solid forms, used in the food or pharmaceutical industry, for example, tablets, are designed for different purposes, among which are (1) to protect the tablet from stomach acids and external contaminants, (2) to protect the stomach from aggressive drug products, (3) provide a delayed release of the active substance, (4) help maintain the shape of the tablet, (5) hide or mask the taste of the drug product, and (6) improve the appearance of the tablet.

In this sense, the coating processes used in the pharmaceutical industry most frequently can be classified into two groups: dragee and film.

Dragee coatings, also referred to in the literature as sugar coatings, are made by applying sugar syrups, usually, sucrose, consisting of sealing, film growth, pigmentation, and polishing steps. These processes can last from 10 to more than 20 hours, the processes require highly specialized labor and have high variability in the quality of the final product. In addition, the weight that the coated cores gain through the dragee process, is normally more than 50% relating to their original weight.

On the other hand, the film coatings are processed by spraying and drying polymer mixtures with additives and pigments previously dispersed in a liquid vehicle and subsequently applied to the solid cores. The processes typically last a few hours. By applying this technique, the weight that the coated cores gain is normally 3 to 5% when the coating is applied to pharmaceutical tablets with the aim of improving the appearance of the final product.

Film coatings are generally preferred because of the shorter time required for applying, better process control, and less specialized labor. However, the brightness of the coated end product, by film coating processes, is typically much lower than the brilliance obtained by dragee processes.

In the state of the art, there are different patent documents in the field of film coatings, which are applied on solid pharmaceutical forms; for example, US patent application 08/466,939 teaches coating product compositions with moisture barrier properties, where the distinctive component is polyvinyl alcohol; in addition in US 09/351,076 there are coating compositions that include polyvinyl alcohol with polyethylene glycol or glycerin as plasticizer, resulting in shimmery gloss, good film adhesion, and good tensile strength.

US 08/778,944 declares compositions for coating products wherein components such as dextrins are included for their formulation.

US patent 08/895,484 explains compositions for glossy white film coatings, based on dextrose with derivatives of the dextrin and starch type, while in WO 2010/052727 low viscosity hypromellose compositions are mentioned, added with starches to achieve compositions of coatings that can be prepared at high solids concentrations of up to 30% in dispersion in water.

Polymer based coatings derived from acrylics and methacrylates as described in WO 2009/086941 and cellulosic derivatives, calcium carbonate as opacifying agent and Xylitol as complexing agent as specified in US 08/241,709 formulations have also been explored in systems of dragee, wherein the addition of the product is made as syrup with the intention of obtaining sugar-free covers.

US Patent 10,159,650 B2 discloses a coating composition where cellulosic derivatives are included as film-forming agents and also includes polyol xylitol as a complexing agent, stating the proportions of use of these components.

Other documents such as WO 2014/024170 mention the use of polyols, in the manufacture of a detergent tablet.

Despite the various efforts that have been made, film coatings for tablets, supplements, pills, lozenges, caplets, or microspheres used in the food or pharmaceutical industries still present technical problems, in particular the finish with reduced brightness.

Therefore, there is a need to have film coatings formulations and processes, which exceed the brightness obtained by currently used film coatings, including those described in the state of the art.

### SUMMARY OF THE INVENTION

The object of the present invention is to use mixtures of materials considered to be safe and suitable for use in drug products, but which until now have not been used in innovative formulations for experimental or commercial products in the manner established in this document.

Another main object of the present invention is to provide a product derived from isomaltulose, which consists of mixing 6-O-α-D-glucopyranosyl-D-mannitol dihydrate with 6-O-α-D-glucopyranosyl-D-sorbitol, also known as Isomalt as a distinctive ingredient in a formulation for obtaining film-shaped coatings for tablets, supplements, pills, lozenges, dragees, caplets or microspheres, used in the food or pharmaceutical industries that exhibit the feature of increased brightness.

A main embodiment of the present invention consists of formulations and processes described herein that include mixing or associating the isomaltulose derivative with film-forming polymers typically used in film coatings, taking as examples, but not limited to, materials selected from the group consisting of: graft copolymer of ethylene glycol and vinyl alcohol, copovidone, polyvinyl alcohol, methacrylates, derivatives thereof and cellulosic derivatives.

Another main embodiment of the present invention is to detail that the association of the isomaltulose derivative, with polymers typically used in film coatings, provides novel advantages in the formation of the applied final film, specifically in the achieved brightness.

Another main object of the present invention is to provide a film-shaped coating for tablets, supplements, pills, lozenges, dragees, caplets or microspheres, used in the food or pharmaceutical industry that have the feature of increased brightness using an inert derivative, non-digestible, without caloric intake or with a very low caloric intake and which is not synthesized in nature by plants or animals in a relevant quantity, with a chemical identity different from carbohydrates such as dextrose, lactose, starch, dextrins and polydextrose.

Another object of the present invention is to provide, as the main film-forming agent for the coating, a copolymer of ethylene glycol and vinyl alcohol, avoiding the use of xylitol or some derived material. In this case, a polyol derived from isomaltulose is used, which is a mixture of 6-O-α-D-glucopyranosyl-D-mannitol dihydrate with 6-O-α-D-glucopyranosyl-D-sorbitol, which is a component not previously used in film coatings, in the manner expressed in the present invention.

Another main object of the present invention is to provide a preparation methodology, with which high concentrations of solids can be achieved, higher than those normally declared in the state of the art and in the generality of methodologies for the preparation of dispersions of film coatings. The preparation methodology consists of dispersing the mentioned innovative formulations in a liquid vehicle, whose advantageous feature is that the viscosities can be less than 0.5 Pa·s with concentrations greater than or equal to 35% solids.

Another main embodiment of the present invention is to provide specific ranges of optimum applying process temperatures, which depend on the composition of the content of the isomaltulose derivative and the content of the film-forming polymer or polymer mixture. The formulations can be processed so that the bed of the tablets can be within a temperature range of 30 to 48 °C

Another main object of the present invention is to provide a film-shaped coating for tablets, supplements, pills, lozenges, dragees, caplets or microspheres, used in the food or pharmaceutical industry that have the feature of increased brightness using an isomaltulose derivative.

An embodiment of the present invention consists of an inert, non-human digestible film-shaped coating, for tablets, supplements, pills, lozenges, dragees, caplets or microspheres, used in the food or pharmaceutical industry exhibiting the feature of increased brightness using an isomaltulose derivative.

Another object of the present invention is to provide a film-shaped coating for tablets, supplements, pills, lozenges, dragees, caplets or microspheres, used in the food or pharmaceutical industry using an isomaltulose derivative having the main feature that the brightness achieved is significantly higher than that obtained with the compositions described in any of the state of the art disclosures.

A further main embodiment of the present invention is that, with the compositions mentioned herein, it is possible to achieve concentrations of dispersions in an aqueous vehicle, significantly higher than typically achievable in film coatings, thereby achieving process times, reduce significantly, with the possibility of completing a cycle of the coating process in half the time compared to formulations containing Hypromellose in its composition. Some proposed formulations are prepared with a concentration of solids greater than or equal to 35%; in contrast, in the state of the art, the maximum achievable concentrations are less than or equal to 35%, typically 8 to 25%.

Yet another main embodiment of the present invention is that some coating films that include the isomaltulose derivative in their composition, have the ability to resist dissolution in non-polar oily media such as vegetable oil and Polaxamer 124.

Also, an embodiment of the present invention is that the coating provides resistance to solubility in oily media, not normally found in pharmaceutical type coatings.

Another embodiment of the invention is the use of the coating in drug products, food supplements or cosmetic products, where the solid coated product is required to remain intact in non-polar media, and at the same time maintain solubility in aqueous media.

### DETAILED DISCLOSURE OF THE INVENTION

The invention disclosed in this document mentions the film coating compositions and the applying procedures with which coated tablets, supplements, pills, lozenges, dragees, caplets or microspheres, used in the food or pharmaceutical industry, can be obtained, by processes recognized as belonging to film coating. The final product obtained with the proposed compositions and procedures consists of a coating for tablets, supplements, pills, tablets, dragees, caplets or microspheres, used in the food or pharmaceutical industry, coated, with finishes that have increased brightness, typically not achievable with film coating compositions and processes described in the state of the art and used to date. The applying procedures can be used in standard coating equipment, such as conventional pans, drilled pans, paddle systems and fluidised beds.

The film coating compositions obtained are based on the incorporation of a product derived from isomaltulose, in the composition of the coating material. The isomaltulose derivative is an appropriate material for pharmaceutical use due to its properties of being innocuous, inert and not digestible or minimally digestible by humans.

The brightness provided by coated compositions by the present invention is one of the fundamental properties that are declared as innovative, presenting measurable and differentiating evidence regarding materials prepared for film coating.

The key and differentiating component is the use of the isomaltulose derivative, which is a semi-synthetic, harmless, inert, non-human digestible polyalcohol, properties that are considered appropriate and desirable for use as a pharmaceutical excipient. This component has not been previously declared as an ingredient in thin layer coating formulations and although it has been declared to be used for dragee systems, film coatings are differentiable from dragee systems as the process times are much shorter, the thickness of the film is very different, the stages of production processes are different and the equipment is different.

Meanwhile, the use of the isomaltulose derivative in tablet cores is mainly cited to achieve fluidity and compactness to powder mixtures for the compression operation of pharmaceutical tablets. The properties imparted by the isomaltulose derivative in the thin layer coating formulas are different from those mentioned above for the manufacture of pharmaceutical tablet cores.

Described below are mixtures of materials useful as film coatings, capable of providing the finished final product with greater brightness, not expected in its intensity, in products of this class.

The invention encompasses coating compositions that include the isomaltulose derivative in pre-mixed manufactured products, known in the pharmaceutical medium as ready-to-use, as well as compositions that include the isomaltulose derivative in the preparation of film coating dispersions, where the addition of the components where the isomaltulose derivative is included, are added sequentially to a solvent or solvent mixture.

The isomaltulose derivative is a mixture of 6-O-α-D-glucopyranosyl-D-mannitol dihydrate with 6-O-α-D-glucopyranosyl-D-sorbitol, which is a component not previously used in film coatings, in the manner expressed in the present invention in which differences are presented in relation to the state of the art such as the declared concentrations of the isomaltulose derivative in the coating, the preparation methodology, wherein with the present invention high concentrations of solids can be achieved, the applying process temperature ranges, such as expressed in the described examples, in order to obtain innovatively increased brightness film coatings for this type of film coating compositions.

Another relevant advantage of the type of innovative coatings described in this document is that the concentration at which they can be prepared in liquid dispersions, prior to their applying by spray, can be significantly higher than that typically prepared by film coatings.

Depending on the composition, there are coatings that can be prepared in a range of 8-12% solids, as an example of those based on hypromellose

In a range of 15-18%, as an example those based on hypromellose-carbohydrate mixtures In a range of 20-25%, as an example those based on polyvinyl alcohol as film-forming

There are reports of up to 35% in compositions including polyvinyl alcohol or ethylene glycol-vinyl alcohol graft copolymers and in low viscosity hypromellose based formulations.

The film-forming polymer or the adhering polymer may come from non-limiting materials such as hypromellose or cellulosic derivatives, polyvinyl alcohol, ethylene glycol-vinyl alcohol graft copolymer, povidone, copovidone.

It is noteworthy as a further contribution in the present invention, that some compositions of film coatings for pharmaceutical use, stated in this document, can be prepared to have solids concentrations higher than 35%, maintaining viscosities below 0.5 Pa·s (500cp), thus decreasing the amount of liquid to evaporate in the process, representing improvements to the preparation concentrations considered for this type of formulations. Within the usual techniques of film coating processes, it is often preferred that the liquid dispersions prepared for its applying have viscosities less than 500cp, so that the dispersion of the liquid dispersion in the coating process is carried out without problems and so that the appearance of the coated tablets has good quality in terms of having a smooth appearance and a uniform finish.

Additionally, it has been found as a relevant additional property of some coating films that include the isomaltulose derivative in their composition, the ability to resist dissolution in non-polar oily media such as vegetable oil and Polaxamero 124. This property of resistance to solubility in oily media is not normally found in pharmaceutical-type coatings and can be useful for use in drug products, food supplements or cosmetic products, where the solid coated product is required to remain intact in non-polar media, but that maintains its properties of being soluble in aqueous media.

To facilitate a better understanding of the present invention, the following examples of certain aspects of some embodiments of the invention are provided. In no way should the following examples be construed to limit, or define, the entire scope of the invention.

Such examples include compositions of solid mixtures, which can be prepared and subsequently dispersed in water as a vehicle, and compositions in which the vehicle is already included in the preparation are also described. The invention is also encompassed in the case that the components are added individually in the preparation of coating dispersions, or as pre-mixes containing the components.

### Example 1. Coating Film Formulation 1

| No. | Description | Composition (%) |
|---|---|---|
| 1 | Isomalt | 45.00 |
| 2 | Copovidone | 10.00 |
| 3 | Polyethylene glycol | 25.00 |
| 4 | Talc | 10.00 |
| 5 | Titanium dioxide | 10.00 |
| Total | | 100.00 |

### Example 2. Coating Film Formulation 2

| No. | Description | Composition (%) |
|---|---|---|
| 1 | Isomalt | 13.50 |
| 2 | Copovidone | 3.00 |
| 3 | Polyethylene glycol | 7.50 |
| 4 | Talc | 3.00 |
| 5 | Titanium dioxide | 3.00 |
| 6 | Water | 70.00 |
| Total | | 100.00 |

### Example 3. Coating Film Formulation 3

| No. | Description | Composition (%) |
|---|---|---|
| 1 | Isomalt | 45.00 |
| 2 | Copovidone | 10.00 |
| 3 | Polyethylene glycol | 25.00 |
| 4 | Talc | 10.00 |
| 5 | Titanium dioxide | 7.65 |
| 6 | Blue Aluminum Lacquer No. 2 | 2.00 |
| 7 | Red Aluminum Lacquer No. 40 | 0.35 |
| Total | | 100.00 |

### Example 4. Coating Film Formulation 4

| No. | Description | Composition (%) |
|---|---|---|
| 1 | Isomalt | 13.500 |
| 2 | Copovidone | 3.000 |
| 3 | Polyethylene glycol | 7.500 |
| 4 | Talc | 3.000 |
| 5 | Titanium dioxide | 2.295 |
| 6 | Blue Aluminum Lacquer No. 2 | 0.600 |
| 7 | Red Aluminum Lacquer No. 40 | 0.105 |
| 8 | Water | 70.000 |
| Total | | 100.000 |

### Example 5. Coating Film Formulation 5

| No. | Description | Composition (%) |
|---|---|---|
| 1 | Isomalt | 22.00 |
| 2 | Hypromellose | 5.00 |
| 3 | Copovidone | 5.00 |
| 4 | Polyethylene glycol | 12.00 |
| 5 | Talc | 50.00 |
| 6 | Titanium dioxide | 6.00 |
| Total | | 100.00 |

### Example 6. Coating Film Formulation 6

| No. | Description | Composition (%) |
|---|---|---|
| 1 | Isomalt | 8.80 |
| 2 | Hypromellose | 2.00 |
| 3 | Copovidone | 2.00 |
| 4 | Polyethylene glycol | 4.80 |
| 5 | Talc | 20.00 |
| 6 | Titanium dioxide | 2.40 |
| 7 | Water | 60.00 |
| Total | | 100.00 |

### Example 7. Coating Film Formulation 7

| No. | Description | Composition (%) |
|---|---|---|
| 1 | Isomalt | 40.00 |
| 2 | Acetyl triethyl citrate | 5.00 |
| 3 | Talc | 25.00 |
| 4 | Titanium dioxide | 29.00 |
| 5 | Blue Aluminum Lacquer No. 2 | 1.00 |
| Total | | 100.00 |

### Example 8. Coating Film Formulation 8

| No. | Description | Composition (%) |
|---|---|---|
| 1 | Isomalt | 12.00 |
| 2 | Talc | 9.00 |
| 3 | Titanium dioxide | 8.70 |
| 4 | Blue Aluminum Lacquer No. 2 | 0.30 |
| 5 | Water | 70.00 |
| Total | | 100.00 |

### Example 9. Coating Film Formulation 9

| No. | Description | Composition (%) |
|---|---|---|
| 1 | Isomalt | 20.00 |
| 2 | Talc | 15.00 |
| 3 | Titanium dioxide | 14.50 |
| 4 | Blue Aluminum Lacquer No. 2 | 0.50 |
| 5 | Water | 50.00 |
| Total | | 100.00 |

### Example 10. Coating Film Formulation 10

| No. | Description | Composition (%) |
|---|---|---|
| 1 | Isomalt | 45.00 |
| 2 | Ethylene glycol-vinyl alcohol graft copolymer | 6.00 |
| 3 | Polyvinyl alcohol | 4.00 |
| 4 | Polyethylene glycol | 25.00 |
| 5 | Talc | 14.0 |
| 6 | Sodium lauryl sulfate | 0.50 |
| 7 | Titanium dioxide | 5.50 |
| Total | | 100.00 |

### Example 11. Coating Film Formulation 11

| No. | Description | Composition (%) |
|---|---|---|
| 1 | Isomalt | 25.00 |
| 2 | Ethylene glycol-vinyl alcohol graft copolymer | 9.00 |
| 3 | Polyvinyl alcohol | 6.00 |
| 4 | Hypromellose | 23.00 |
| 5 | Polyethylene glycol | 15.00 |
| 6 | Talc | 9.24 |
| 7 | Titanium dioxide | 7.00 |
| 8 | Yellow Aluminum Lacquer No. 6 | 5.00 |
| 9 | Red Aluminum Lacquer No. 40 | 0.76 |
| Total | | 100.00 |

### Example 12. Coating Film Formulation 12

| No. | Description | Composition (%) |
|---|---|---|
| 1 | Isomalt | 20.00 |
| 2 | Ethylene glycol-vinyl alcohol graft copolymer | 35.00 |
| 3 | Polyethylene glycol | 13.00 |
| 4 | Kaolin | 16.00 |
| 5 | Titanium dioxide | 15.00 |
| 6 | Yellow iron oxide | 0.50 |
| 7 | Red iron oxide | 0.50 |
| Total | | 100.0 |

### Example 13. Coating Film Formulation 13

| No. | Description | Composition (%) |
|---|---|---|
| 1 | Isomalt | 20.00 |
| 2 | Ethylene glycol-vinyl alcohol graft copolymer mixed with polyvinyl alcohol | 12.00 |
| 3 | Hypromellose | 18.00 |
| 4 | Polyethylene glycol | 12.00 |
| 5 | Talc | 17.00 |
| 6 | Titanium dioxide | 16.00 |
| 7 | Yellow Aluminum Lacquer No. 10 | 5.00 |
| Total | | 100.00 |

### Example 14. Coating Film Formulation 14

| No. | Description | Composition (%) |
|---|---|---|
| 1 | Isomalt | 6.00 |
| 2 | Ethylene glycol-vinyl alcohol graft copolymer mixed with polyvinyl alcohol | 3.60 |
| 3 | Hypromellose | 5.40 |
| 4 | Polyethylene glycol | 3.60 |
| 5 | Talc | 5.10 |
| 6 | Titanium dioxide | 4.80 |
| 7 | Yellow Aluminum Lacquer No. 10 | 1.50 |
| 8 | Water | 70.00 |
| Total | | 100.00 |

### Example 15. Coating Film Formulation 15

| No. | Description | Composition (%) |
|---|---|---|
| 1 | Isomalt | 24.00 |
| 2 | Ethylene glycol-vinyl alcohol graft copolymer mixed with polyvinyl alcohol | 12.00 |
| 3 | Hypromellose | 12.00 |
| 4 | Polyethylene glycol | 12.00 |
| 5 | Talc | 18.00 |
| 6 | Titanium dioxide | 18.00 |
| 7 | Carmine aluminum lacquer | 4.00 |
| Total | | 100.00 |

### Example 16. Coating Film Formulation 16

| No. | Description | Composition (%) |
|---|---|---|
| 1 | Isomalt | 45.00 |
| 2 | Ethylene glycol-vinyl alcohol graft copolymer mixed with polyvinyl alcohol | 10.00 |
| 3 | Polyethylene glycol | 25.00 |
| 4 | Talc | 14.00 |
| 5 | Titanium dioxide | 4.00 |
| 6 | Sodium lauryl sulfate | 0.50 |
| 7 | Red Aluminum Lacquer No. 40 | 1.50 |
| Total | | 100.00 |

### Example 17. Coating Film Formulation 17

| No. | Description | Composition (%) |
|---|---|---|
| 1 | Isomalt | 18.00 |
| 2 | Ethylene glycol-vinyl alcohol graft copolymer mixed with polyvinyl alcohol | 4.00 |
| 3 | Polyethylene glycol | 10.00 |
| 4 | Talc | 5.60 |
| 5 | Titanium dioxide | 1.60 |
| 6 | Sodium lauryl sulfate | 0.20 |
| 7 | Red Aluminum Lacquer No. 40 | 0.60 |
| 8 | Water | 60.00 |
| Total | | 100.00 |

The percentages described in Examples 1 to 17 are defined by weight relating to the total weight of the composition.

In pharmaceutical type film coating processes, it is generally preferred to prepare coverage dispersions for coating not exceeding 0.5 Pa·s (500 cp) in order that, in the applying process, the spraying of the material is carried out without problems to obtain smooth and aesthetically acceptable coated tablets. The table in Example 18 shows the viscosities of a series of coatings in common use and also of some coatings that include Isomalt in their composition. It is evident that some formulations containing Isomalt, can allow its preparation in concentrations from 35% of solids content or higher.

In the table of Example 18, the viscosities were determined with a Brookfield type viscometer, brand W&J Instrument Co. Model RV-2M. Considering that the dispersions of coatings in general are non-Newtonian type fluids, wherein the measured viscosity depends on the measurement conditions, the needle and the speed of rotation of the needle are shown in square brackets when making the respective measurement.

### Example 18. Film Coating Product Viscosity Table

| Polymeric Base in examples of film coatings, in typical formulations | Viscosity (mPa s) at various coverage preparation concentrations/[needle, speed in rpm] | | | | | | | |
|---|---|---|---|---|---|---|---|---|
| | 10% | 12% | 15% | 20% | 25% | 30% | 35% | 40% |
| Hypromellose | 120 / [2,12] | **240** / [2,12] | 680 / [2,12] | | | | | |
| Hypromellose + Polydextrose | 130 / [1,30] | 140 / [2,30] | **408** / [2,30] | 1050 / [3,30] | | | | |
| Hypromellose + Lactose | 90 / [1,30] | 150 / [2,30] | **290** / [2,30] | 1210 / [2,12] | | | | |
| Hypromellose + Xylitol | 70 / [1,30] | 100 / [1,30] | **290** / [2,30] | 660 / [2,30] | | | | |
| Polyvinyl alcohol | | | 140 / [1,12] | **340** / [2,12] | 600 / [2,12] | | | |
| Ethylene glycol and vinyl alcohol graft copolymer | | | | 80 / [1,12] | **190** / [1,12] | 1160 / [2,12] | | |
| Ethylene glycol and vinyl alcohol graft copolymer with polyvinyl alcohol | | | | 70 / [1,12] | **340** / [1,12] | 680 / [2,12] | | |
| Ethylene glycol and vinyl alcohol graft copolymer with polyvinyl alcohol + Hypromellose + **Isomalt** | | | | 70 / [1,60] | 120 / [2,60] | 170 / [2,60] | **440** / [2,60] | 1020 / [3,30] |
| Ethylene glycol and vinyl alcohol graft copolymer with polyvinyl alcohol + **Isomalt** | | | | 20 / [1,60] | 30 / [1,60] | 40 / [1,60] | 100 / [1,30] | **160** / [1,30] |
| Copovidone + **Isomalt** | | | | | | 50 / [1,30] | 60 / [1,30] | **90** / [1,30] |

As a relevant property of the film coating compositions that the present invention includes, there is the high brightness of the films that are obtained by using isomalt as an ingredient in its formulation. The table in Example 19 shows the brightness of films with formulations that do not include Isomalt, compared to the brightness of compositions with film-forming polymers supplemented with Isomalt. Measurements were made with the brand KSJ Glossmeter instrument, model MG6-SA with 60° angle of incidence. It is evident that the inclusion of isomalt promotes increased brightness in films, increasing on average by 15 GU, increasing brightness by more than 300%. In order that the comparisons were objective without intervention of the color factor, the brightness was determined on white films. In the same way, with the aim of making the comparisons impartial, without the intervention of the polishing factor that exists during the coating processes caused by the action of friction between the tablets, the brightness was measured in films applied on inert substrate such as white cardboard. Brightness measurements are reported in Gloss Units (GU).

### Example 19. Comparison Tables of Applied Film Gloss Measurement

| Polymer of reference | Brightness excluding Isomalt (GU) | Brightness including Isomalt (GU) |
|---|---|---|
| Mixture of ethylene glycol and vinyl alcohol graft copolymer with polyvinyl alcohol | 7.2 | 18.3 |
| Hypromellose | 8.3 | 26.0 |
| Polyvinyl alcohol | 5.3 | 21.3 |

| Polymer of reference | Brightness including Xylitol (GU) | Brightness including Isomalt (GU) |
|---|---|---|
| Mixture of ethylene glycol and vinyl alcohol graft copolymer with polyvinyl alcohol | 6.0 | 18.3 |

The table in Example 20 shows the brightness of composition films where only one film-forming agent is involved. It is appreciated that the Isomalt component alone generates greater brightness, however, when comparing with the results in Table 19, a synergy effect is observed where the brightness of coatings of film-forming mixtures mixed with Isomalt is greater than the brilliance generated by any of the individually tested film-forming agents.

### Example 20. Comparison Table of Applied Film Gloss Measurement

| Film-forming agent of reference | Brightness (GU) |
|---|---|
| Polyvinyl alcohol | 5.3 |
| Hypromellose | 8.3 |
| Mixture of ethylene glycol and vinyl alcohol graft copolymer, with polyvinyl alcohol | 7.2 |
| Isomalt | 16.5 |

Optimal coating processing conditions, i.e. those required to apply a thin layer film to tablets, granules, pellets, or capsules, are dependent on the composition of the film. In the case of compositions that include Isomalt as an ingredient, it has been found that, in some cases, the optimal applying temperature ranges are wider, meaning this is an advantage, given that many substrates due to instability or various causes, can require processing at relatively low temperatures. The table in Example 21 shows optimal applying temperature ranges for various types of film coating formulations. It is noteworthy that some of the compositions that include Isomalt can be applied at moderately lower temperatures than traditional coatings.

### Example 21. Comparison table of optimal process temperatures in coating applying. Tablet bed temperature

| Coating product | Optimal applying temperatures (tablet bed temperature) |
|---|---|
| Hypromellose base in water | 40 - 45 °C |
| Hypromellose base with carbohydrates | 38 - 45 °C |
| Polyvinyl Alcohol Base in Water | 38 - 44 °C |
| Ethylene glycol and vinyl alcohol graft base | 38 - 44 °C |
| Ethylene glycol and vinyl alcohol graft base plus polyvinyl alcohol, plus hypromellose plus Isomalt (composition of example 14) | 30 - 48 °C |

As used in this document, the term "increased brightness" refers to the gloss shown by the coating film and is in the range of 10 to 30 or more Gloss Units (GU), measured with a brand KSJ Glossmeter instrument, model MG6-SA with 60° angle of incidence.

In this document, the terms "Isomalt" or "isomaltulose derivative" are used interchangeably and refer to the mixture of 6-O-α-D-glucopyranosyl-D-mannitol dihydrate with 6-O-α-D-glucopyranosyl- D-sorbitol.

The term film-forming polymer is used interchangeably as film-forming or adherent polymer to denote polyvinyl alcohol, hypromellose, ethylene glycol and vinyl alcohol graft copolymer, with polyvinyl alcohol, copovidone, polyvinyl alcohol, methacrylate derivatives, isomalt and cellulosic derivatives

### Additional observations

Therefore, the present invention is well adapted to achieve the aforementioned purposes and advantages, as well as those inherent therein. The particular embodiments disclosed above are illustrative only, as the present invention can be modified and practiced in different but equivalent apparent ways for those skilled in the art who have the benefit of the teachings herein. Furthermore, no limitation is intended to the details of the features shown in this document, in addition to those described in the claims that follow. It is therefore evident that the particular illustrative embodiments disclosed above can be altered, combined or modified and that all such variations are considered within the scope and spirit of the present invention.

The invention disclosed illustratively herein can be suitably practiced in the absence of any item not specifically disclosed herein and/or any optional item disclosed herein. While the formulations and methods are described in terms of "comprising", "containing", or "including" different components or steps, the formulations and methods may also "consist essentially of" or "consist of" the different components and steps. The terms formulation, formulations, composition and compositions are used interchangeably and refer to the same. All the numbers and ranges disclosed above may vary by some amount. Whenever a numerical range with a lower limit and an upper limit is disclosed, any number and any included range that falls within the range is specifically disclosed. The terms in the claims have their simple, ordinary meaning unless explicitly and clearly defined otherwise by the patent holder. Furthermore, the indefinite article "one", as used in the claims, is defined herein to refer to one or more than one of the elements you introduce. If there are any conflicts in the uses of a word or term in this specification and one or more patents or other documents that may be incorporated herein by reference, the definitions that are consistent with this description should be adopted.

## Claims

1. A coating composition for solid dosage forms, selected from the group consisting of tablets, supplements, pills, lozenges, dragees, caplets or microspheres, comprising:
a) An isomaltulose derivative;
b) Adherent polymers; and
c) Individual or combined additives.

2. The composition according to claim **1,** wherein the isomaltulose derivative is in a percentage from 5% to 85% by weight, relating to the total weight of the composition.

3. The composition according to claim **1,** wherein the isomaltulose derivative is preferably in a percentage from 15% to 50% by weight, relating to the total weight of the composition.

4. The composition according to claim **1,** wherein adherent polymers are selected from the group consisting of hypromellose, cellulosic derivatives, copovidone, povidone, methacrylate derivatives, polyvinyl alcohol, ethylene glycol and vinyl alcohol graft copolymer, ethylene glycol and vinyl alcohol graft copolymer or combinations thereof.

5. The composition according to claim **1,** wherein the adherent polymers are in a percentage from 5% to 60% by weight, relating to the total weight of the composition.

6. The composition according to claim **1,** wherein the adherent polymers are preferably in a percentage from 10% to 40% by weight, relating to the total weight of the composition.

7. The composition according to claim **1,** wherein the individual or combined additives have functions of opacifying plasticizers or pigments from 5% to 60% by weight, relating to the total weight of the composition.

8. The composition according to claim 1, wherein the individual or combined additives are preferably from 0.1% to 40% by weight, relating to the total weight of the composition.

9. The composition according to claim **1,** wherein the final coated product shows increased brightness, ranging from 10 to 30 or more Gloss Units (GU), measured at 60° angle of incidence.

10. The composition according to claim **1,** wherein the brightness of coatings of adherent polymer mixtures with isomaltulose derivatives is significantly greater than the brightness generated by any of the individually tested adherent polymers.

11. The composition according to claim **10,** wherein the brightness is increased from 100% to 400% and between 10 and 18 GU relating to the brightness of the individually tested adherent polymers.

12. The composition according to claim **1,** wherein it is used in an inert coating, in film-shaped for tablets, supplements, pills, lozenges, dragees, caplets or microspheres, used in the food, cosmetic or pharmaceutical industries having the feature of increased brightness, using isomalt.

13. The composition according to claim **1,** wherein it is used in the preparation of a dispersion in an aqueous vehicle, with concentrations from 8% to 50% by weight of the composition relating to the total weight of the dispersion.

14. The composition according to claim **13,** wherein the preferred concentration of the preparation can be greater than or equal to 35% by weight of the composition relating to the total weight of the dispersion.

15. The composition according to claims **1** to **14,** wherein it has a viscosity of less than 0.5 Pa s (500cp) in its preparation.

16. The composition according to claims **1** to **15,** when applied, reduced process times are needed, due to the decrease in the amount of liquid to evaporate in the process.

17. The composition according to claims **1** to **16,** wherein one of the options for preparing the dispersion in water comprises the following formulation:
| | |
|---|---|
| Isomalt | 6.00 (%) |
| Hypromellose | 5.40 (%) |
| Ethylene glycol-vinyl alcohol graft copolymer mixed with polyvinyl alcohol | 3.60 (%) |
| Polyethylene glycol | 3.60 (%) |
| Talc | 5.10 (%) |
| Titanium dioxide | 4.80 (%) |
| Pigment | 1.50 (%) |
| Water | 70.00 (%) |
| Total | 100.00 (%) |

18. he composition according to claims **1** to **17,** wherein it can be applied and dried on cores with a tablet bed temperature in a range of 30 °C to 48 °C.

19. The composition according to claims **1** to **16,** wherein one of the options for preparing the dispersion in water comprises the following formulation:
| | |
|---|---|
| Isomalt | 18.00 (%) |
| Ethylene glycol-vinyl alcohol graft copolymer mixed with polyvinyl alcohol | 4.00 (%) |
| Polyethylene glycol | 10.0 (%) |
| Talc | 5.60 (%) |
| Titanium dioxide | 1.60 (%) |
| Sodium lauryl sulfate | 0.20 (%) |
| Pigment | 0.60 (%) |
| Water | 60.00 (%) |
| Total | 100.00 (%) |

20. The composition according to claims **1** to **19,** wherein it can be prepared at a concentration of 40% solids or more, it can form a film with high brightness, where the brightness is greater than 15 Gloss Units measured with a KSJ brand Glossmeter instrument, MG6-SA model with 60° angle of incidence.

21. A coating composition for solid dosage forms, selected from the group comprising tablets, supplements, pills, lozenges, dragees, caplets or microspheres, wherein comprises:
d) An isomaltulose derivative;
e) Adherent polymers; and
f) Individual or combined additives.
wherein the composition has the ability to resist dissolution in non-polar oily media, selected from the group comprising: vegetable oil and Polaxmer 124.

22. The use of the composition according to claims **1** to **21,** in drug products, food supplements or cosmetic products, wherein the solid coated product is required to remain intact in non-polar media, and at the same time maintain solubility in aqueous media.

23. The composition according to any one of the previous claims, wherein the components can be added individually in the preparation of aqueous or non-aqueous coating dispersions.

24. The composition according to any one of the previous claims, wherein the components can be added as pre-mixes containing the components in the preparation of aqueous or non-aqueous coating dispersions.

25. The composition according to any one of the previous claims, wherein the components can be added sequentially or non-sequentially to a solvent or solvent mixture.
